# EUROPEAN PATENT SPECIFICATION

(11) **EP 3 471 783 B1**
(45) Date of publication and mention of the grant of the patent: **26.04.2023**
(21) Application number: 17732160.1
(22) Date of filing: 14.06.2017
(51) Int. Cl.: A61L 9/01, A61L 9/12, A61L 9/14

(54) **ATOMISER SYSTEM FOR DISPENSING A FRAGRANCE**
ZERSTÄUBERSYSTEM ZUR AUSGABE EINES DUFTSTOFFES
SYSTÈME ATOMISEUR POUR LA DIFFUSION D'UN PARFUM

(30) Priority: 17.06.2016 GB 201610656
(43) Date of publication of application: 24.04.2019
(73) Proprietor: Reckitt Benckiser (Brands) Limited, Slough, Berkshire SL1 3UH (GB)
(72) Inventor: BASSINDALE, Philip, Hull East Yorkshire HU8 7DS (GB); BIRD, Arron, Cheshire Greater Manchester SK15 1HW (GB); BRUNT, James, Salt Lake City 84104 (US); CREES, James, Hull East Yorkshire HU8 7DS (GB); DAS, Avijit, Gurgaon 122001 Haryana (IN)
(74) Representative: Soellner, Siegfried
(86) International application number: PCT/GB2017/051723
(87) International publication number: WO 2017/216548

(56) References cited:
- WO-A1-2010/134164
- US-A- 4 304 688
- US-A1- 2009 101 730
- US-A1- 2011 318 276
- US-B1- 6 446 880
- Chemicals Idemitsu Kosan Co. Ltd: "Idemitsu Kosan Co.,Ltd", , 8 March 2016 (2016-03-08), XP055727047, Retrieved from the Internet: URL:http://www.idemitsu-chemicals.de/files /datasheets/broschures/PerformanceChemical s/IPSolvent.pdf [retrieved on 2020-09-02]

## Description

### Background to the invention

Ultrasonic atomisers have long been used to vapourise liquid fragrance compositions to freshen the air. They offer advantages over alternative methods (heated liquid electricals/candles for example) in that they do not require heating to operate. Which means they are safer, no naked flames and they use much less power than heated emanators, so they can be readily run with batteries for extended periods. This gives them flexibility of placement.

Such devices usually comprise a vibrating plate, a source of power, a reservoir of liquid fragrance and a means to transport the liquid fragrance to the plate for atomisation.

Examples of such devices in the prior art are included in documents JP3342095, US2008041972 A1, US2001042794A, US6446880 B1 and US6341732 B1. Problems associated with these devices include obtaining consistent and reliable emanation and leaks. The contact between the piezo vibrating plate and the source of the liquid fragrance must be in finely balanced to allow for successful operation. Drop out and un-atomised spray of the liquids is common when the plates are not vibrating optimally.

WO 2010/134164 A1 discloses a method for pest insect control, and an ultrasound pest insect control device.

US 6446880 B1 discloses a replaceable reservoir for an atomizing apparatus.

US 2011/318276 A1 discloses an aerosol composition with enhanced dispersion effects.

US 4304688 A discloses a liquid aromatic deodorizing composition.

### Summary of the invention

The present invention provides an atomiser for dispensing a fragrance in accordance with claim 1, a method of fragrancing the air in accordance with claim 9 and a use in accordance with claim 10.

### Description

Piezo devices and systems for fragrancing the air in the art suffer from a variety of inefficiencies. Poorly aligned interfaces between the vibrating plates and the source of liquid fragrance to be atomised cause many difficulties. As does the rate at which fluid is delivered to the plate from the wick. A low fluid delivery rate means poor fragrance release rate and a high delivery rate gives rise to spray formation (incomplete atomisation).

It has surprisingly been found that a lot of these issues can be resolved by the use of particular solvents as carrier fluids in the fragrancing liquids to be atomised. Liquid fragrance compositions containing high concentrations of branched or iso-alkanes seem to provide excellent atomising properties and delivery properties. Low levels of spray (large particle sizes) and very low residues (particle drop out) are obtained when high levels of branched alkane solvents are used in the carrier liquids.

Fragrance compositions containing at least 50% branched alkanes in the carrier solvent have superior dispensing characteristics in ultrasonic atomising dispensers.

For the purposes of the present invention branched alkanes means any alkanes that are not completely linear. They may have single or multiple branching. Preferred alkanes are iso-alkanes.

The branched alkanes of the present invention comprise mixtures (or single compounds) with between 11 and 16 carbon atoms.

Commercial examples of useful branched alkanes include the Isopar^{™} class of products from Exxon mobile. A particularly advantageous branched chain solvent for use in the present invention is Isopar M^{™}

Preferably the branched alkane carrier solvent has a boiling point of between 240 and 270 °C, preferably between 250 and 260 degrees °C and most preferably between 252 and 258 degrees °C.

Preferably the branched alkane carrier solvent has a molecular weight of between 160 and 200, preferably between 185 and 195 and most preferably 188.

Preferably the carrier solvent consists substantially of branched chain alkanes.

The fragrancing liquid of the present invention comprises
a) Fragrance composition 2-25% by weight and
b) Carrier solvent 75 -98% by weight.

Preferably the liquid comprises at least 5% by weight of fragrance composition, preferably at least 10% by weight fragrance composition and most preferably at least 15% by weight fragrance composition.

The liquid comprises 75-98% by weight carrier solvent, more preferably at least 80% and most preferably at least 85% by weight of carrier solvent.

The liquid transport member is a key part of the device. For successful operation the liquid transport member and orifice plate have to interact successfully. The liquid transport member may be made from any suitable material. Preferably the liquid transport member moves liquid from the reservoir to the plate by wicking or capillary action.

The liquid transport member of the present invention is a wick comprising paper, cotton, nylon, or polypropylene. Most preferably the wick will be formed from polypropylene.

Preferably the wick is between 4cm and 14 cm in length, more preferably between 5cm and 12 cm and most preferably between 6 and 10 cm in length.

Preferably the atomiser is powered by battery. Preferably the atomiser comprises at least one battery, and more preferably at least two batteries.

The atomiser may require between 1 and 12 volts to operate, more preferably 3- 4.5 volts.

The atomiser may be provided with control circuitry to allow for regular activation of the device over time. Settings may be provided to control length of activation and time between activations. This allows the device to be left in the home or office and provide a constant release of fragrance over many weeks and days.

Activations of the orifice plate to release atomised liquid fragrance composition may last between 1 second and 30 seconds, preferably between 2 seconds and 15 seconds, more preferably between 3 seconds and 10 seconds.

Timing between activations may be between 2 mins and 30 mins, preferably between 5 mins and 20 mins, most preferably between 7 mins and 15 mins.

The skilled person can vary these parameters depending on the intensity of the fragrance desired, strength of fragrance used and concentration of the fragrance composition within the fragrance liquid.

A second aspect to the present invention involves a method of fragrancing the air comprising activating the device of the first aspect of the invention.

This may be manually or automatically by setting up dispensing parameters. Automatic dispensing will require control circuits to activate the device at intervals.

A third aspect of the present invention is the use of a separate refill of fragrance as the liquid reservoir in the device of the first aspect of the invention.

This will comprise a container suitable for standalone sale.

Preferably this will be a small flask or bottle. This may be made of any material. Preferably this will be made of glass or plastic.

This will preferably be readily insertable into the atomiser device of the present invention. This should be such that the user may easily remove empty refills. The user may also decide to replace a partially used refill for a refill of a differing fragrance if desired.

The fragrance refill will comprise
1) a liquid; further comprising A fragrance composition and a carrier solvent; and
2) a wick, extending from inside the refill to the outside;
wherein the carrier solvent comprises at least 50% by weight of C11-C16 branched chain alkanes.

Including the wick with the refills prevents the mixing of fragrances in the wick. A rapid fragrance change can be carried out without blending different fragrances.

Wicks can get clogged in time. Using a new wick with each refill prevents this from happening.

The refill may comprise at least 5% by weight of fragrance composition, preferably at least 10% by weight fragrance composition and most preferably at least 15% by weight fragrance composition.

The refill liquid comprises 75-98% by weight carrier solvent, more preferably at least 80% and most preferably at least 85% by weight of carrier solvent.

The carrier solvent may comprise at least 60% by weight of branched alkanes, preferably at least 70% by weight of branched alkanes and most preferably at least 90% by weight branched alkanes.

The carrier solvent of the refill may consist entirely of branched-alkanes.

The branched alkane carrier solvent may have a boiling point of between 240 and 270 °C, preferably between 250 and 260 °C and most preferably between 252 and 258 degrees °C.

The branched alkane carrier solvent may have a molecular weight of between 170 and 200, preferably between 185 and 195 and most preferably 188.

A particularly preferred refill contains:
15% by weight of fragrance composition; and
85% by weight of a branched alkane solvent.

More preferably the branched alkane solvent comprises Isopar-M ^{®}.

A device comprising the refill above and a polypropylene wick has been shown to yield excellent fragrance dispersion characteristics with virtually no residues remaining.

A device comprising the refill above and a polypropylene wick has been shown to yield excellent fragrance dispersion characteristics with virtually no residues remaining.

## Claims

1. An atomiser for dispensing a fragrance; the atomiser comprising
a) an outer housing;
b) an orifice plate and piezo electric element;
c) a liquid reservoir, comprising a liquid to be atomised, the liquid comprising a fragrance composition and a carrier solvent; and
d) a liquid transport member;
wherein the liquid transport member extends from within the liquid reservoir to abut the orifice plate;
wherein the liquid transport member is a wick comprising paper, cotton, nylon, or polypropylene and is substantially contained within the liquid reservoir and both the wick and liquid reservoir are readily detachable together from the atomiser;
wherein the liquid comprises:
a) a fragrance composition 2-25% by weight and
b) a carrier solvent 75-98% by weight; and
wherein the carrier solvent comprises at least 50% by weight C11-C16 branched chain alkanes.

2. The atomiser of claim 1 wherein the liquid transport member is a wick comprising polypropylene.

3. The atomiser of claim 1 or 2 wherein the wick is between 4cm and 14 cm in length, more preferably between 5cm and 12 cm and most preferably between 6 and 10 cm in length.

4. The atomiser of any of the previous claims wherein the liquid comprises at least 5% by weight of fragrance composition, preferably at least 10% by weight fragrance composition and most preferably at least 15% by weight fragrance composition, and/or wherein the liquid comprises at least 80% by weight of carrier solvent and most preferably at least 85% by weight of carrier solvent.

5. The atomiser of any of the previous claims wherein the carrier solvent is greater than 99% by weight a branched alkane solvent, preferably an iso-alkane or isoparaffin solvent.

6. The atomiser of any of claims 1 to 5 wherein the branched alkane carrier solvent has a boiling point of between 240 and 270 °C, preferably between 250 and 260 °C and most preferably between 252 and 258 °C.

7. The atomiser of any of the previous claims wherein the branched alkane carrier solvent has a molecular weight of between 170 and 200, preferably between 185 and 195 and most preferably 188.

8. The atomiser of any of the previous claims wherein the atomiser comprises at least one battery, preferably at least two batteries, and/or requires between 1 and 12 volts to operate, more preferably 3- 4.5 volts, and/or comprises control circuitry to allow for regular activation of the device over time.

9. A method of fragrancing the air comprising activating the device of claims 1-8.

10. The use of a refill as the liquid reservoir in the device of any one of claims 1-8, the refill comprising;
a) a liquid; further comprising 2-25% of a fragrance composition and 75 -98% of a carrier solvent; and
b) a wick comprising paper, cotton, nylon, or polypropylene, the wick extending from inside the refill to the outside;
wherein the carrier solvent comprises at least 50% by weight of C11-C16 branched chain alkanes.

11. The use according to claim 10 wherein the liquid comprises at least 5% by weight of fragrance composition, preferably at least 10% by weight fragrance composition and most preferably at least 15% by weight fragrance composition, and/or wherein the liquid comprises at least 80% by weight of carrier solvent and most preferably at least 85% by weight of carrier solvent.

12. The use according to any one of claims 10 or 11 wherein the carrier solvent comprises at least 60% by weight of branched alkanes, preferably at least 70% by weight of branched alkanes and most preferably at least 90% by weight branched alkanes, preferably the carrier solvent consists of branched-alkanes.

13. The use according any one of claims 10 to 12 wherein the branched alkane carrier solvent has a boiling point of between 240 and 270 degrees C, preferably between 250 and 260 degrees C and most preferably between 252 and 258 degrees C.

14. The use according to any one of claims 10 to 13 wherein the branched alkane carrier solvent has a molecular weight of between 170 and 200, preferably between 185 and 195 and most preferably 188.

## Patentansprüche

1. Zerstäuber zur Ausgabe eines Duftstoffes; wobei der Zerstäuber umfasst
a) ein äußeres Gehäuse;
b) eine Öffnungsplatte und ein piezoelektrisches Element;
c) ein Flüssigkeitsreservoir, das eine zu zerstäubende Flüssigkeit umfasst, wobei die Flüssigkeit eine Duftstoffzusammensetzung und ein Trägerlösungsmittel umfasst; und
d) ein Flüssigkeitstransportelement;
wobei sich das Flüssigkeitstransportelement von innerhalb des Flüssigkeitsreservoirs erstreckt, um an der Öffnungsplatte anzuliegen;
wobei das Flüssigkeitstransportelement ein Docht ist, der Papier, Baumwolle, Nylon oder Polypropylen umfasst und im Wesentlichen innerhalb des Flüssigkeitsreservoirs enthalten ist, und sowohl der Docht als auch das Flüssigkeitsreservoir leicht zusammen vom Zerstäuber abnehmbar sind;
wobei die Flüssigkeit umfasst:
a) eine Duftstoffzusammensetzung in 2-25 Gew.-% und
b) ein Trägerlösungsmittel in 75-98 Gew.-%; und
wobei das Trägerlösungsmittel mindestens 50 Gew.-% verzweigtkettige C11-C16-Alkane umfasst.

2. Zerstäuber nach Anspruch 1, wobei das Flüssigkeitstransportelement ein Docht ist, der Polypropylen umfasst.

3. Zerstäuber nach Anspruch 1 oder 2, wobei der Docht zwischen 4 cm und 14 cm lang ist, bevorzugter zwischen 5 cm und 12 cm und am meisten bevorzugt zwischen 6 und 10 cm lang ist.

4. Zerstäuber nach einem der vorhergehenden Ansprüche, wobei die Flüssigkeit mindestens 5 Gew.-% Duftstoffzusammensetzung, vorzugsweise mindestens 10 Gew.-% Duftstoffzusammensetzung und am meisten bevorzugt mindestens 15 Gew.-% Duftstoffzusammensetzung umfasst, und/oder wobei die Flüssigkeit mindestens 80 Gew.-% Trägerlösungsmittel und am meisten bevorzugt mindestens 85 Gew.-% Trägerlösungsmittel umfasst.

5. Zerstäuber nach einem der vorhergehenden Ansprüche, wobei das Trägerlösungsmittel mehr als 99 Gew.-% von einem verzweigten Alkanlösungsmittel, vorzugsweise einem Isoalkan- oder Isoparaffinlösungsmittel ist.

6. Zerstäuber nach einem der Ansprüche 1 bis 5, wobei das verzweigte Alkanträgerlösungsmittel einen Siedepunkt zwischen 240 und 270 °C, vorzugsweise zwischen 250 und 260 °C und am meisten bevorzugt zwischen 252 und 258 °C aufweist.

7. Zerstäuber nach einem der vorhergehenden Ansprüche, wobei das verzweigte Alkanträgerlösungsmittel ein Molekulargewicht zwischen 170 und 200, vorzugsweise zwischen 185 und 195, und am meisten bevorzugt 188 aufweist.

8. Zerstäuber nach einem der vorhergehenden Ansprüche, wobei der Zerstäuber mindestens eine Batterie, vorzugsweise mindestens zwei Batterien umfasst und/oder zwischen 1 und 12 Volt, bevorzugter 3-4,5 Volt zum Betrieb benötigt, und/oder eine Steuerschaltung umfasst, um eine regelmäßige Aktivierung der Vorrichtung im Laufe der Zeit zu ermöglichen.

9. Verfahren zum Beduften der Luft, das das Aktivieren der Vorrichtung nach den Ansprüchen 1-8 umfasst.

10. Verwendung einer Nachfüllpatrone als Flüssigkeitsreservoir in der Vorrichtung nach einem der Ansprüche 1-8, wobei die Nachfüllpatrone umfasst:
a) eine Flüssigkeit; die ferner 2-25 % einer Duftstoffzusammensetzung und 75-98 % eines Trägerlösungsmittels umfasst; und
b) einen Docht, der Papier, Baumwolle, Nylon oder Polypropylen umfasst, wobei sich der Docht von der Innenseite der Nachfüllpatrone nach außen erstreckt;
wobei das Trägerlösungsmittel mindestens 50 Gew.-% verzweigtkettige C11-C16-Alkane umfasst.

11. Verwendung nach Anspruch 10, wobei die Flüssigkeit mindestens 5 Gew.-% Duftstoffzusammensetzung, vorzugsweise mindestens 10 Gew.-% Duftstoffzusammensetzung und am meisten bevorzugt mindestens 15 Gew.-% Duftstoffzusammensetzung umfasst, und/oder wobei die Flüssigkeit mindestens 80 Gew.- % Trägerlösungsmittel und am meisten bevorzugt mindestens 85 Gew.- % Trägerlösungsmittel umfasst.

12. Verwendung nach einem der Ansprüche 10 oder 11, wobei das Trägerlösungsmittel mindestens 60 Gew.- % verzweigte Alkane, vorzugsweise mindestens 70 Gew.-% verzweigte Alkane und am meisten bevorzugt mindestens 90 Gew.-% verzweigte Alkane umfasst, wobei vorzugsweise das Trägerlösungsmittel aus verzweigten Alkanen besteht.

13. Verwendung nach einem der Ansprüche 10 bis 12, wobei das verzweigte Alkanträgerlösungsmittel einen Siedepunkt zwischen 240 und 270 Grad C, vorzugsweise zwischen 250 und 260 Grad C und am meisten bevorzugt zwischen 252 und 258 Grad C aufweist.

14. Verwendung nach einem der Ansprüche 10 bis 13, wobei das verzweigte Alkanträgerlösungsmittel ein Molekulargewicht zwischen 170 und 200, vorzugsweise zwischen 185 und 195 und am meisten bevorzugt 188 aufweist.

## Revendications

1. Atomiseur pour la diffusion d'un parfum ; l'atomiseur comprenant
a) un boîtier extérieur ;
b) une plaque à orifice et un élément piézoélectrique ;
c) un réservoir de liquide comprenant un liquide à atomiser, le liquide comprenant une composition parfumée et un solvant support ; et
d) un élément de transport de liquide ;
dans lequel l'élément de transport de liquide s'étend depuis l'intérieur du réservoir de liquide pour venir buter contre la plaque à orifice ;
dans lequel l'élément de transport de liquide est une mèche comprenant du papier, du coton, du nylon ou du polypropylène et est sensiblement contenu dans le réservoir de liquide et à la fois la mèche et le réservoir de liquide sont facilement détachables ensemble de l'atomiseur ;
dans lequel le liquide comprend :
a) de 2 % à 25 % en poids d'une composition parfumée et
b) de 75 % à 98 % en poids d'un solvant support ; et
dans lequel le solvant support comprend au moins 50 % en poids d'alcanes à chaîne ramifiée en C11-C16.

2. Atomiseur selon la revendication 1, dans lequel l'élément de transport de liquide est une mèche comprenant du polypropylène.

3. Atomiseur selon la revendication 1 ou 2, dans lequel la mèche a une longueur comprise entre 4 cm et 14 cm, plus préférablement entre 5 cm et 12 cm et idéalement entre 6 cm et 10 cm.

4. Atomiseur selon l'une quelconque des revendications précédentes, dans lequel le liquide comprend au moins 5 % en poids de composition parfumée, de préférence au moins 10 % en poids de composition parfumée et idéalement au moins 15 % en poids de composition parfumée, et/ou dans lequel le liquide comprend au moins 80 % en poids de solvant support et idéalement au moins 85 % en poids de solvant support.

5. Atomiseur selon l'une quelconque des revendications précédentes, dans lequel le solvant support est constitué à plus de 99 % en poids d'un solvant à base d'alcanes ramifiés, de préférence d'un solvant à base d'iso-alcanes ou d'isoparaffines.

6. Atomiseur selon l'une quelconque des revendications 1 à 5, dans lequel le solvant support à base d'alcanes ramifiés a un point d'ébullition compris entre 240 °C et 270 °C, de préférence entre 250 °C et 260 °C et idéalement entre 252 °C et 258 °C.

7. Atomiseur selon l'une quelconque des revendications précédentes, dans lequel le solvant support à base d'alcanes ramifiés a un poids moléculaire compris entre 170 et 200, de préférence entre 185 et 195 et idéalement de 188.

8. Atomiseur selon l'une quelconque des revendications précédentes, l'atomiseur comprenant au moins une batterie, de préférence au moins deux batteries, et/ou nécessitant entre 1 volt et 12 volts pour fonctionner, plus préférablement entre 3 volts et 4,5 volts, et/ou comprenant une circuiterie de commande pour permettre une activation régulière de l'appareil dans le temps.

9. Procédé permettant de parfumer l'air, comprenant l'activation du dispositif selon les revendications 1 à 8.

10. Utilisation d'une recharge comme réservoir de liquide dans le dispositif selon l'une quelconque des revendications 1 à 8, la recharge comprenant :
a) un liquide ; comprenant en outre entre 2 % et 25 % d'une composition parfumée et entre 75 % et 98 % d'un solvant support ; et
b) une mèche comprenant du papier, du coton, du nylon ou du polypropylène, la mèche s'étendant de l'intérieur de la recharge vers l'extérieur ;
dans laquelle le solvant support comprend au moins 50 % en poids d'alcanes à chaîne ramifiée en C11-C16.

11. Utilisation selon la revendication 10, dans laquelle le liquide comprend au moins 5 % en poids de composition parfumée, de préférence au moins 10 % en poids de composition parfumée et idéalement au moins 15 % en poids de composition parfumée, et/ou dans laquelle le liquide comprend au moins 80 % en poids de solvant support et idéalement au moins 85 % en poids de solvant support.

12. Utilisation selon l'une quelconque des revendications 10 ou 11, dans laquelle le solvant support comprend au moins 60 % en poids d'alcanes ramifiés, de préférence au moins 70 % en poids d'alcanes ramifiés et idéalement au moins 90 % en poids d'alcanes ramifiés, de préférence le solvant support étant constitué d'alcanes ramifiés.

13. Utilisation selon l'une quelconque des revendications 10 à 12, dans laquelle le solvant support à base d'alcanes ramifiés a un point d'ébullition compris entre 240 degrés C et 270 degrés C, de préférence entre 250 degrés C et 260 degrés C et idéalement entre 252 degrés C et 258 degrés C.

14. Utilisation selon l'une quelconque des revendications 10 à 13, dans laquelle le solvant support à base d'alcanes ramifiés a un poids moléculaire compris entre 170 et 200, de préférence entre 185 et 195 et idéalement de 188.
